# FASCICULE DE BREVET EUROPEEN

(11) **EP 0 387 154 B1**
(45) Date de publication et mention de la délivrance du brevet: **28.04.1993**
(21) Numéro de dépôt: 90400628.5
(22) Date de dépôt: 08.03.1990
(51) Int. Cl.: A61K 49/00, A61K 49/02, A61K 9/12, A61K 47/48

(54) **Compositions aérosols destinées à l'imagerie, au diagnostic et à la thérapie ciblée de foyers inflammatoires et tumoraux**
Aerosol-Zusammensetzung für Bilderzeugungs-Diagnostik und Therapie von Krebs- und Infektionsherden
Aerosol compositions for the imaging, diagnosis and targetted therapy of inflamed and tumoral focuses

(30) Priorité: 08.03.1989 FR 8903034
(43) Date de publication de la demande: 12.09.1990
(73) Titulaire: PIERRE FABRE MEDICAMENT, 92100 Boulogne (FR)
(72) Inventeur: Dussourd D'Hinterland, Lucien, F-81100 Castres (FR); Le Pape, Alain, F-37130 Langeais (FR); Normier, Gérard, F-81100 Castres (FR); Pinel, Anne-Marie, F-81100 Castres (FR); Durand, Jacques, F-81100 Castres (FR)
(74) Mandataire: Warcoin, Jacques

(56) Documents cités:
- EP-A- 0 246 149
- EP-A- 0 288 382
- S.T.N. INTERNATIONAL, Karlsruhe, Base de C.A.S., CA, vol. 76, 1972, résumé no. 110934j, Columbus, Ohio, US; S.J. ENNA et al.: "Absorption of saccharides and urea from the rat lung"
- S.T.N. INTERNATIONAL, Karlsruhe, Base de C.A.S., CA, vol. 107, 1987, résumé no. 190558m, Columbus, Ohio, US; U. RAMSTADT: "Stimulation of NK cells using Klebsiella pneumoniae membrane proteoglycans"

## Description

La présente invention concerne des compositions aérosols comportant un agent vecteur capable de reconnaître et se fixer sur des foyers inflammatoires et tumoraux mobilisant dans leur environnement proche des macrophages, et un substrat aérosol.

Plus particulièrement, la présente invention concerne des compositions aérosols pour l'imagerie médicale ou le diagnostic in vivo ou ex vivo et des kits comprenant de telles compositions et des compositions pour la thérapie ciblée des foyers inflammatoires et tumoraux mobilisant dans leur environnement proche des macrophages.

Selon leur caractéristique essentielle, les compositions selon la présente invention comportent un agent vecteur consistant en un composé polysaccharidique choisi parmi le composé D 25, le composé D 25 oxydé dans lequel le résidu galacto furanose (Gal_{f}) de la chaîne polysaccharidique linéaire du D 25 a été transformée en arabinose, et leurs dérivés de type amide, ester ou éther, ainsi que leurs sels et dérivés d'ammonium quaternaires, ainsi que leurs dérivés obtenus par greffage de phosphatides.

Le produit dénommé D 25 est un composé polysaccharidique extrait des protéoglycanes membranaires bactériens composé essentiellement d'unité galactose et ayant un poids moléculaire de 30 ± 10 kD. Ce polysaccharide a été décrit dans le brevet français n° 86 06765.

Il était présenté comme possédant des propriétés immunostimulantes, notamment vis-à-vis de l'induction d'interféron endogène et de l'activation des cellules NK (Natural Killers). Ce composé polysaccharidique est préférentiellement isolé d'une souche mutante non capsulée et non pathogène de Klebsiella pneumoniae biotype a, déposée à la Collection Nationale de l'Institut Pasteur sous le n° 145-I-IP.

La structure du D 25 comporte, d'une part une chaîne polysaccharidique linéaire constituée par répétition environ 5 fois d'un motif monomérique de 10 sucres, et, d'autre part, d'une séquence de liaison unique, plus complexe, à laquelle sont liées de courtes chaînes peptidiques.

L'unité monomérique répétitive de la chaîne polysaccharidique linéaire ne renferme que du galactose sous forme pyrane et furane dans les proportions suivantes :

3 β Gal p, 3 α Gal p, 2 β Gal f, 2 α Gal f.

La séquence de cette unité monomérique est la suivante : 
[ PS ] = ... —[→ β Gal p ——→ β Gal f ——→ α Gal p ——→ β Gal p ——→
1,3 1,3 1,3
α Gal f ——→ α Gal p ——→ α Gal f ——→ β Gal p ——→ α Gal p ——→ β Gal f —]→
1,3 1,3 1,3 1,3 1,3 1,3 n
structure de liaison

La structure unique de liaison est fixée à l'extrémité de la chaîne polysaccharidique linéaire. Elle contient des résidus glucose, galactose, glucosamine, heptose et acide mannodéoxyoctulosonique. De courtes chaînes peptidiques sont liées à cette structure.

La séquence probable est la suivante :

→α Glc p NH2 →α Glc p →α Gal p →α Hep p →α Hep p → Man Oc. A
1,3 1,3 1,4 1,3 1,3 1,5
↑
3,1
α Glc p

Les amino acides suivants composant les deux chaînes peptidiques associées sont en nombre suivant :
Acide aspartique : 3
Acide glutamique : 2
Sérine : 1
Proline : 1
Glycine : 1,5
Alanine : 2
Valine : 1
Leucine : 1
Lysine : 1

### Abréviations :

- β Gal p: = β Galactopyranose
- α Gal p: = α Galactopyranose
- β Gal f: = β Galactofuranose
- α Gal f: = α Galactofuranose
- α Glc p: = α Glucopyranose
- α Glc p, NH₂: = α Glucosamine
- α Hep p: = α Heptose (D. Mannoheptose)
- Man Oc. A: = 3-deoxy-D-manno-octulosonic acid

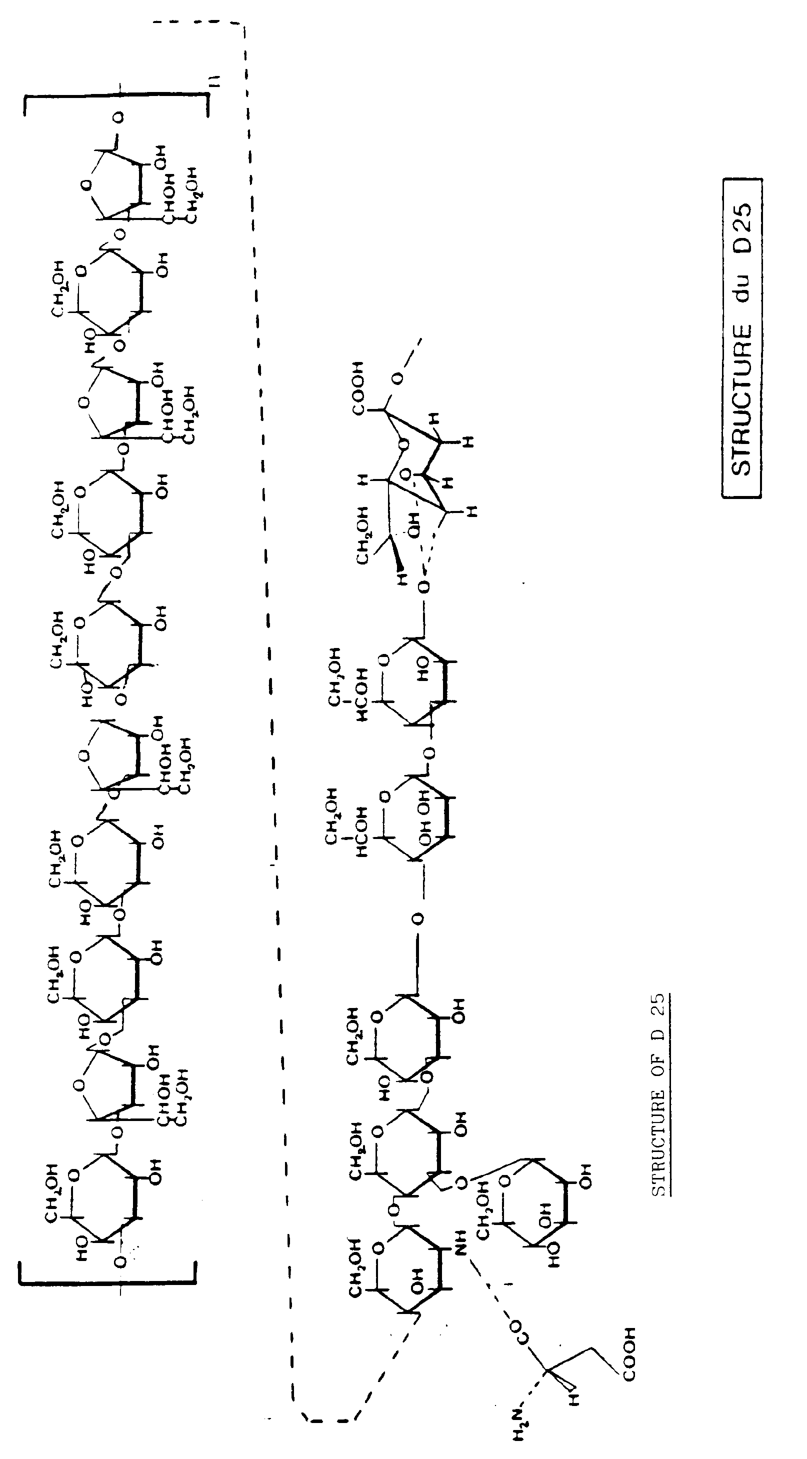

STRUCTURE OF D 25

STRUCTURE du D25

Les dérivés du D 25 de type amide, ester ou éther du D 25 ainsi que les sels et dérivés d'ammonium quaternaire selon la présente invention sont des dérivés d'hémisynthèse de ce composé, décrits dans la demande de brevet n° 86 06765 ainsi que dans la demande de brevet n° 87 05690.

Parmi ces composés, on peut citer les dérivés du D 25 avec des acides, des amines ou des alcools a chaîne grasse, c'est-à-dire présentant au moins 4 atomes de carbone alicyclique.

Les dérivés du D 25 oxydé selon la présente invention sont également ceux dans lesquels au moins une partie des résidus galacto furannose (Gal_{f}) de la chaîne polysaccharidique linéaire du D 25 ont été transformés en arabinose, sans aucune autre modification du produit initial.

Parmi ces composés, l'un d'entre eux est particulièrement intéressant, il s'agit du composé dans lequel tous les résidus galactofurannose de la chaîne polysaccharidique linéaire ont été transformés en arabinose et qui est défini par le monomère suivant : 
→[→ β Gal p → β Ara → α Gal p → β Gal p → α Ara
1,3 1,3 1,3 1,3 1,3
→ α Gal p → α Ara → β Gal p → α Gal p → β Ara →]→···
1,3 1,3 1,3 1,3 1,3 1,3
dans lequel Gal p signifie galacto pyrannose (formes A et B)

Ara signifie arabinose (formes α et β).

Il s'agit également des dérivés choisis parmi les amides, les esters, les éthers ou les dérivés d'ammonium quaternaire avec une amide, un acide ou un alcool de ces composés oxydés du D 25.

Les dérivés du D 25 sont également obtenus par greffage de phosphatides sur le D 25 ou ses dérivés de type amide, ester, éther, ainsi que les sels de dérivé de l'ammonium quaternaire, et les dérivés oxydés. Ils peuvent être obtenus à partir de phosphatides tels que les lécithines d'oeuf ou de soja, lécithines hydrogénées, azolectines, céphalines, sphingosine, sphingomyéline, cette liste n'étant pas exhaustive et pouvant être élargie à d'autres types de phospholipides portant des fonctions amino ou carboxy libres.

Le composé D 25 et ses dérivés se sont avérés présenter une affinité pour les macrophages et en faire plus particulièrement un agent vecteur idéal pour reconnaître et se fixer sur les foyers inflammatoires et tumoraux mobilisant des macrophages.

Comme il sera vu par la suite, une caractéristique essentielle des compositions selon la présente invention est qu'elles sont administrées par voie aérosol. Les essais poursuivis ont en effet démontré que toute autre voie d'administration que la voie aérosol ne conduisait à aucun résultat satisfaisant.

Il convient en outre de remarquer que de façon tout à fait avantageuse le composé D 25 ou ses dérivés administrés par inhalation selon la présente invention, ne nécessite pas d'avoir recours à l'utilisation de forme galénique et notamment ne nécessite pas d'être encapsulé dans des liposomes pour être administré par voie aérosol.

Les propriétés physico-chimiques du produit du type amphiphile permettent son adsorption pulmonaire directe.

Le D 25 et ses dérivés se sont avérés présenter une affinité pour les cellules du type monocytes-macrophages qui en font un agent vecteur idéal pour reconnaître et se fixer sur les foyers inflammatoires et tumoraux mobilisant ces cellules.

De façon appropriée donc, les compositions aérosols selon la présente invention seront destinées plus particulièrement à l'imagerie ou au diagnostic in vivo ou ex vivo pour la mise en évidence de foyers inflammatoires et tumoraux mobilisant dans leur environnement proche des macrophages. On peut mettre en évidence des foyers pathologiques de taille millimétrique.

Ledit composé polysaccharidique de la composition choisie parmi le D 25 et ses dérivés peut être alors marqué par un élément détectable tel qu'un élément radioactif, paramagnétique ou fluorescent.

Ledit composé polysaccharidique de la composition peut aussi être détectable indirectement, c'est-à-dire qu'il peut être détecté via une substance elle-même marquée par un élément détectable tel que mentionné précédemment, ladite substance reconnaissant et se liant spécifiquement auxdits composés polysaccharidiques. A titre de substance reconnaissant et se liant spécifiquement auxdits composés polysaccharidiques, on peut citer un anticorps dressé contre ledit composé.

Les kits d'imagerie ou de diagnostic in vivo ou ex vivo selon l'invention comporteront donc soit une composition dans laquelle ledit composé polysaccharidique est marqué, soit une composition dans laquelle ledit composé n'est pas marqué et une seconde composition qui peut être injectable comportant une substance reconnaissant ledit composé polysaccharidique, ladite substance étant elle-même marquée.

On peut citer comme élément radioactif approprié selon l'invention un radionucléide détectable par scintigraphie tel que le technetium Tc^{99m}, ou encore à titre non limitatif l'iode¹²³ et l'Indium¹¹¹.

Les compositions aérosols selon la présente invention se sont révélées tout particulièrement efficaces pour l'imagerie et le diagnostic in vivo par scintigraphie.

Le radionucléide peut être détectable par visualisation ou par comptage per opératoire par une sonde guidée manuellement. Il s'agira alors d'un diagnostic par comptage et non d'imagerie.

Compte tenu de sa senbilité, l'imagerie scintigraphique est, dans de nombreux cas, la meilleure technique permettant la visualisation de foyers tumoraux ou inflammatoires de taille inférieure au centimètre. Elle est généralement basée sur le ciblage de cellules pathologiques, par exemple à l'aide de drogues reconnues spécifiquement par un récepteur, ou d'anticorps monoclonaux spécifiques d'antigènes cellulaires, dont la clairance tissulaire doit être rapide pour permettre un bon contraste scintigraphique à partir d'un nombre minimal de cellules ciblées, actuellement de l'ordre de 5 à 7.10⁸. Ces contraintes limitaient généralement jusqu'à maintenant les possibilités de visualisation pour de petites tumeurs seulement détectables par le dosage de leurs marqueurs tumoraux, par exemple les antigènes carcinoembryonnaires, Ca15.3, Ca19.9.

Selon l'invention, une autre statégie pour la mise en évidence de foyers pathologiques de petite taille est proposée prenant en compte l'existence très précoce au cours de la croissance tumorale d'un recrutement au niveau de la tumeur elle-même comme dans son proche environnement de cellules immunocompétentes et principalement de macrophages (EVANS R. Biochim. Biophys. Acta 1986, 865 : 1-11 ; WEI W.Z. et al Biochim. Biophys. Acta 1986, 865 : 13-26 - BUGESLKI P.J. et al Am. J. Pathol. 1985, 118 419:424). La possibilité d'une visualisation scintigraphique des tumeurs par le ciblage des macrophages péritumoraux via des anticorps marqués dirigés contre des antigènes de différenciation, par exemple l'antigène MAC 1 correspondant au récepteur du C3bi, a été tentée récemment dans le cas de la tumeur de Lewis greffée chez la souris C57BL/6 (PELLEN P. et al. Immunobiol. 1986, 173 : 197-198). Cette technique souffre cependant des limites de l'immunoscintigraphie pour laquelle la fixation non spécifique élevée due à la faible clairance tissulaire de l'anticorps ne permet pas d'obtenir un contraste scintigraphique suffisant pour la visualisation de tumeurs de taille millimétrique (CHATAL J.F. Nucl. Med. Bio., 1986, 13 : 203-205).

L'amélioration des performances scintigraphiques d'un ligand capable de reconnaître in vivo des cellules immunocompétentes est donc très dépendante de la vitesse de son épuration tissulaire.

Or, précisément l'agent vecteur selon l'invention consistant dans le D 25 et ses dérivés non seulement s'est avéré capable de s'associer principalement à des cellules du système des phagocytes mononucléés, tels que les monocytes du sang mais encore présente une vitesse d'épuration tissulaire élevée permettant d'obtenir un contraste scintigraphique suffisant pour la visualisation de tumeurs de tailles millimétriques.

On notera que ces résultats ont conduit à évaluer l'efficacité du ciblage de ce type cellulaire, in vivo, après injection intraveineuse chez le singe Babouin de D 25 et de ses dérivés préalablement marqués par un radioisotope émetteur gamma tel que le 99 m Technetium qui est bien adapté à la détection scintigraphique. Dans les 10 minutes qui suivent l'injection, plus de 80 % du produit injecté est piégé dans le foie et la rate, très vraisemblablement par suite de la séquestration, par les cellules du système réticuloendothélial, des formes polymériques de ces composés. La voie intraveineuse est donc inutilisable. Cependant, compte tenu du caractère amphiphile de ces molécules et de la masse moléculaire de leurs unités monomériques de 30 kD, une absorption au niveau de la barrière alvéolocapillaire du poumon se produit, permettant de ce fait une administration systémique du principe actif (réf. : ENNA S.J. et SCHANKER L.S., Am. J. Physiol 1972, 223 : 1227-31. C'est pourquoi une caractéristique essentielle des compositions selon l'invention est leur administration par voie aérosol.

Dans des conditions optimisées d'aérosolisation, le passage sanguin des molécules marquées est effectif en 120 ± 40 minutes avec une demi-vie sanguine de l'ordre de 140 minutes, l'élimination ayant lieu par voie urinaire. Durant les 6 premières heures consécutives à l'administration, l'absence d'image scintigraphique de la thyroïde permet d'affirmer l'absence de dissociation du traceur Tc^{99m} qui, à l'état libre, ayant le même rayon ionique que l'ion iodure est rapidement accumulé dans les cellules thyroïdiennes. Un modèle de pathologie expérimentale de réaction inflammatoire évolutive ganglionnaire et parenchymateuse pulmonaire a été développé par inhalation de Béryllium métal chez le singe pour évaluer l'aptitude des composés à cibler in vivo les cellules immunocompétentes de ces lésions.

Dans ces conditions expérimentales, des ganglions péritrachéaux et médiastinaux de petite taille, le plus souvent inférieure à 0,5 cm à l'autopsie, ont été visualisés dans les 3 heures suivant l'administration d'un radioaérosol de D 25 ou de dérivé de D 25 oxydé ou d'un autre dérivé obtenu par couplage covalent de phosphatide au D 25. A l'examen histologique, les ganglions fixants étaient caractérisés par une infiltration de cellules macrophagiques chargées de particules associée à une déplétion lymphoïde para corticale et folliculaire.

Cette fixation n'est pas liée au simple drainage via les ganglions trachéobronchiques (réf. : HARMSEN A.G., Science, 1985, 230 : 1277-80) puisque les scintigraphies témoins réalisées dans les mêmes conditions avec des liposomes unilamellaires radiomarqués au 99m Technetium ne permettent pas d'obtenir d'image ganglionnaire, y compris dans des étapes plus tardives de l'examen.

Dans le cas des animaux présentant des granulomes pulmonaires confirmés à l'examen anatomopathologique, une fixation du D 25 et de ses dérivés est observée d'une manière constante dans le territoire pathologique alors que les techniques scintigraphiques et radiologiques conventionnelles (tomodensitométrie X, Imagerie par Résonance Magnétique Nucléaire) n'ont pas été capables de révéler de telles lésions.

L'évaluation en pathologie humaine de l'activité scintigraphique du D 25 et de ses dérivés a été entreprise après vérification de l'absence de pyrogène et un contrôle d'innocuité chez la souris, le rat et le singe Papio-papio. Les scintigraphies ont été réalisées chez des patients porteurs de tumeurs pulmonaires métastasées ou non et non antérieurement traités ou de sarcoïdose avec atteinte ganglionnaire médiastinale connue. Ces examens ont été pratiqués dans le Service de Médecine Nucléaire du Centre Hospitalier Universitaire de Tours, après obtention du consentement informé des patients. Pour éviter tout risque d'accident d'hypersensibilisation, les examens n'ont été pratiqués qu'une seule fois sur chaque patient, et aucun effet secondaire, tant clinique que biologique n'a été observé. Les résultats obtenus, confrontés à ceux des autres examens d'Imagerie Médicale, et, chez les sujets opérés, aux données histologiques, montrent une fixation intense du D 25 et de ses dérivés technétiés deux à quatre heures après l'inhalation, au niveau des ganglions médiastinaux des sujets porteurs de sarcoïdose et des complexes gangliotumoraux de divers types de tumeurs pulmonaires.

Dans un mode de réalisation particulier, les compositions ou kits d'imagerie ou diagnostic selon l'invention sont utilisées pour la détection de la sarcoïdose, des alvéolites, des granulomatoses, des pneumocystoses, des pathologies interstitielles du poumon et des lésions inflammatoires et infectieuses.

Compte tenu des propriétés amphiphile du D 25 et de la rapidité de son absorption par la barrière alvéolocapillaire du poumon, les compositions en kit diagnostic selon l'invention peuvent être utilisées pour l'exploration fonctionnelle isotopique pulmonaire. Les fonctions de ventilation et d'absorption seront étudiées par une réalisation particulière, le produit étant administré pendant une acquisition scintigraphique dynamique par exemple à raison d'une image par minute pendant l'inhalation (ventilation) puis pendant la première demi-heure de l'absorption pulmonaire (détermination de la clairance permettant l'estimation de l'intégrité de la barrière alvéolocapillaire).

Le D 25 ou ses dérivés, marqué directement ou détectable indirectement par un radionucléide et administré par inhalation sont également utilisés selon la présente invention pour la détection et la mesure per opératoire de l'activité des foyers fixants dans le cadre du bilan d'extension des pathologies inflammatoires ou tumorale.

On peut également citer selon la présente invention une utilisation du D 25 ou de ses dérivé administré par inhalation pour l'étude par l'immunofluorescence, par exemple avec un anticorps anti D 25, de l'étendue de la réaction histiocytaire et des limites des foyers inflammatoires et tumoraux en histopathologie.

Enfin, la présente invention a également pour objet des compositions aérosols pour la thérapie des inflammations et tumeurs mobilisant des macrophages dans leur environnement, caractérisées en ce qu'elles comportent ledit composé polysaccharidique choisi parmi le D 25 et ses dérivés, couplé à un principe actif anti-inflammatoire ou antitumoral respectivement, administrable par voie aérosol.

D'autres avantages et caractéristiques de la présente invention apparaîtront dans les exemples qui vont suivre.

Dans les exemples qui vont suivre,
- La figure 1 représente la scintigraphie au D 25, thorax face postérieure, 3 h après inhalation du babouin n° 444, 3 mois après induction d'une bérylliose pulmonaire.

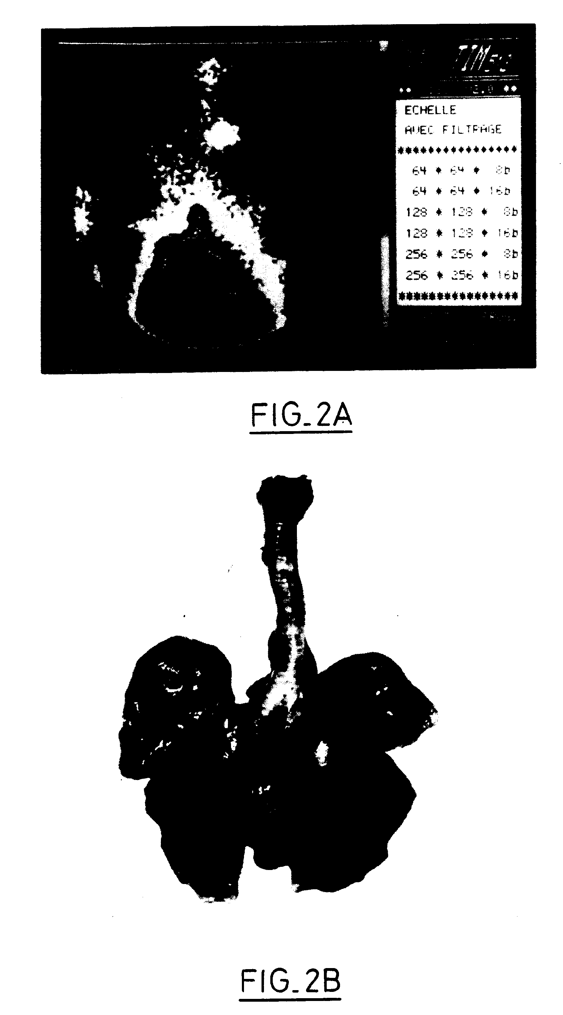

- La figure 2a représente la scintigraphie au D 25 au stade évolué de la maladie, le contrôle anatomique étant présenté sur la figure 2b.
- Les figures 3a et 3b représentent chez un patient présentant une sarcoïdose de stade I avec une augmentation de la taille des ganglions thoraciques et des foyers réticulonodulaires, l'imagerie scintigraphique des ganglionshilaires avec le D 25, 4 heures après inhalation (figure 3a) et celle du Gallium pratiquée 48 heures après l'injection i.v. (figure 3b).
- Les figures 4a et 4b représentent un patient présentant une sarcoïdose de stade II correspondant à une pathologie pulmonaire diffuse sans anomalie ganglionnaire, la scintigraphie au D 25 des poumons (figure 4a) et la scintigraphie au Gallium (figure 4b).
- La figure 5 représente la scintigraphie au D 25 4 heures après inhalation d'une sarcoïdose de stade III évoluée au stade de la fibrose avec lésions inflammatoires mais sans ganglion.
- La figure 6 représente la scintigraphie au D 25 thorax face postérieure 4 h 30 après inhalation, le patient présentant des métastases pulmonaires bilatérales.
- La figure 7 représente la scintigraphie au D 25 4 heures après inhalation du thorax en face postérieure, le patient présentant un carcinome du lobe inférieur gauche.
- La figure 8 représente la scintigraphie au D 25 4 heures après inhalation d'une localisation ganglionnaire d'un patient présentant un adénocarcinome à grandes cellules du lobe supérieur du poumon droit.
- La figure 9 représente la scintigraphie au D 25 (cuisses face antérieure), 4 heures après inhalation d'une métastase sous forme cutanée de taille inférieure à 5 mm dans la région antérointerne de la cuisse droite d'un patient présentant une métastase d'un adénocarcinome du poumon gauche.

### EXEMPLE 1 PROCEDE INDUSTRIEL D'OBTENTION DU D 25

Le D 25 est isolé de la membrane d'une souche non capsulée et non pathogène de Klebsiella pneumoniae, biotype a, déposée sous le n° 145.I.IP à la collection de l'Institut Pasteur de PARIS.

Un procédé industriel, spécialement développé pour la production à grande échelle du D 25 est décrit ci-après :

### 1.1 Obtention de lysats bactériens clarifiés

La biomasse de Klebsiella pneumoniae est obtenue par culture en fermenteur en milieu liquide dans des conditions classiques. La seule contrainte spécifique étant l'arrêt brusque de la croissance par refroidissement à + 4°C en fin de phase exponentielle de croissance pour préserver l'intégrité des structures biologiques.

La biomasse est séparée du milieu de culture par centrifugation continue à froid sur séparateur industriel de type Sharples ou Wesfalia. Après lavage par remise en suspension dans du sérum physiologique stérile et centrifugation, le concentrat cellulaire est stocké congelé en attendant d'être traité.

Le concentrat bactérien est décongelé dans un réacteur et mis en suspension dans du tampon tris-HCL (10 mM) pH 7,0 contenant MgCl₂ (10 mM) et NaCl (0,15 M) à 4°C pour avoir une concentration finale équivalente à 50 g de cellules sèches par litre de suspension. On ajoute ensuite 5 mg de DNase par litre de suspension.

Les cellules microbiennes sont ensuite désintégrées par passage en continu sur des broyeurs industriels de type APV Manton Gaulin. Le lysat bactérien ainsi obtenu est soumis à une première clarification continue à 15.000 xg sur séparateur Sharples à 4°C pour éliminer les résidus de broyage et les germes non broyés. Le culot de centrifugation est éliminé et le surnageant recueilli, il constitue le lysat clarifié.

### 1.2 Obtention du protéoglycane membranaire de Klebsiella pneumoniae

Le lysat clarifié obtenu précédemment est acidifié par l'acide acétique à pH 4,2 ± 0,2 et laissé 30 minutes au repos à + 4°C. Le précipité d'impuretés formé est éliminé par centrifugation continue sur Sharples à 15.000 xg. Le surnageant limpide contenant le protéoglycane membranaire est neutralisé puis dialysé avec de l'eau distillée par ultrafiltration sur membrane coupant à 10.000 daltons, pour avoir une résistivité égale ou supérieure à 1.000 Ω cm⁻¹.

### 1.3 Obtention du D 25

Le protéoglycane membranaire est un polymère de haut poids moléculaire (> 1,5 x 10⁶ daltons). Il est dépolymérisé par une hydrolyse alcaline ménagée pendant 1 Heure à 56°C dans l'hydroxyde de sodium 0,5 M, puis refroidi et neutralisé.

Les protéines et fractions de parois contaminantes sont ensuite éliminées par une digestion enzymatique dans les conditions suivantes :

On ajoute à la suspension du Tris qsp 10 mM, de l'EDTA qsp 4 mM puis on ajuste le pH à 7,0. On ajoute ensuite 0,1 g/l de protéinase K et 0,1 g/l de lysozyme et l'on incube 2 heures à 37°C sous agitation.

Le D 25 est ensuite purifié par deux précipitations consécutives avec 2 volumes d'alcool éthylique à - 20°C en présence d'acétate de sodium 0,5 M, le précipité obtenu après 30 minutes de repos étant recueilli par centrifugation continue à 15.000 xg.

Le précipité final de D 25 est repris dans l'eau distillé à 4°C et la solution clarifiée par centrifugation à 30.000 xg pendant 60 minutes. Le surnageant est recueilli puis dialysé avec de l'eau distillée jusqu'à une résistivité égale ou supérieure à 2.500 Ω cm⁻¹ par ultrafiltration sur membrane coupant à 10.000 daltons.

Le dialysat ainsi obtenu est stérilisé par filtration sur membrane 0,22 µm puis lyophilisé stérilement. Ce lyophilisat constitue le D 25.

Chaque opération industrielle est réalisée sur des échantillons de biomasse correspondant à 2 à 4 kg de cellules sèches et permet d'obtenir de 40 à 100 g de D 25 pur.

### EXEMPLE 2 OBTENTION DES DERIVES D'HEMISYNTHESE DU D 25

2.1 Des immunomodulateurs obtenus par hémisynthèse à partir du D 25 ont déjà été décrits dans le brevet n° 86 06765 du 2 Mai 1986.
2.2 Un dérivé du D 25 oxydé dans lequel les galactofuranoses de la chaîne polysaccharidique ont été convertis en arabinoses a également été décrit dans la demande de brevet n° 87 05690 du 22 Avril 1987.
   Une solution de D 25 purifié est diluée afin d'obtenir 20 g de polysaccharide par litre de solution. On ajoute alors de l'acétate de sodium 0,1 M et le pH est ajusté à 3,8.
   On ajoute ensuite 15 g de métaperiodate de sodium par litre de solution puis on maintient sous agitation pendant 48 heures à l'obscurité et à température 15°C.
   L'excès de métaperiodate est ensuite éliminé par précipitation avec de l'hydroxyde de Baryum sous forme d'une solution concentrée ajoutée progressivement sous agitation jusqu'à fin de précipitation. Le précipité ainsi formé est éliminé par simple filtration.
   Au filtrat précédent, on ajoute alors 21,6 g de NaBH₄ puis on laisse réagir pendant 18 heures à température ambiante. L'excès de NaBH₄ est ensuite détruit par addition d'acide acétique jusqu'à neutralité.
   La solution obtenue est dialysée, concentrée sur membrane coupant à 10 000 Daltons puis lyophilisée. Le lyophilisat ainsi obtenu constitue le dérivé de D 25 oxydé.
2.3 De nouveaux dérivés obtenus par greffage de phosphatides sur le D 25 possédant des propriétés originales notamment au niveau de leur pharmacocinétique et de leur absorption intestinale ont été créés.

Ils peuvent être obtenus à partir de phosphatides tels que lécithines d'oeuf ou de soja, lécithines hydrogénées, azolectine, céphalines, sphingosine, sphingomyéline, cette liste n'étant pas exhaustive et pouvant être élargie à d'autres types de phospholipides portant des fonctions amino ou carboxy libres.

### Couplage d'une lécithine hydrogénée au D 25

2.3.1. Activation du D 25 par l'EDAC (1-éthyl-3-(3-Diméthylaminopropyl) carbodiimide hydrochloride)
   100 mg de D 25 sont dissous dans 10 ml d'eau distillée, puis on ajoute 25 mg d'EDAC préalablement dissous dans 2 ml d'eau distillée et le pH de la réaction est ajusté à 4,75.
2.3.2. Après 30 minutes d'agitation à pH 4,75, une solution de 100 mg de lécithine hydrogénée (lécinol S.10 Noorden) dans 10 ml de THF est ajoutée et le mélange réactionnel est gardé sous agitation toute la nuit.
2.3.3. Le mélange réactionnel est ensuite évaporé sous vide et le résidu obtenu est extrait 3 fois par le chloroforme pour éliminer l'excès de lécithine n'ayant pas réagi.
2.3.4. Après extraction au chloroforme le résidu est repris dans l'eau distillée puis dialysé et lyophilisé.

Le taux de lécithine couplée au D 25 est ensuite déterminé par dosage spectrophotométrique avec le kit diagnostic Boehringer.

Dans les conditions expérimentales ci-dessus décrites il est de 5.8%.

En agissant sur l'excès de lécithine dans le milieu réactionnel, il est possible de moduler le caractère hydrophobe des dérivés obtenus.

### EXEMPLE 3 RADIOMARQUAGE DU D 25 ET DE SES DERIVES

Dans un flacon type pénicilline sous vide d'air, 1 mg de produit lyophilisé est dissous dans 1 ml de solution isotonique de NaCl tamponnée à pH 7,2 par des phosphates ou par du Tris 0,01 M préalablement dégazée sous vide. La solution est ensuite transférée, sans introduction d'air, dans un autre flacon de même type contenant 195 µg de chlorure stanneux fraîchement lyophilisé (SIN, M.S. et al. J. Nucl. Med, 1971, 12 : 204-208) à partir d'une solution mère préparée en HCl 2 N, sous vide.

Après 10 minutes de réduction sous agitation intermittente, le marquage est réalisé par addition, sans introduction d'air, de 1110 à 1480 MBq de technétium 99m sous forme de Pertechnetate de sodium fraîchement élué d'un générateur (CEA, ORIS). Le volume final est ajusté à 4 ml par addition de la solution isotonique tamponnée à pH 7,2 et dégazée.

En 15 minutes à 18-20°C sous agitation intermittente, le marquage est total avec un rendement, contrôlé par radio HPLC sur colonne TSK 2000, supérieur à 99,5 %.

Dans le flacon de marquage, à l'abri de l'air, la liaison Tc-D 25 est stable pendant 1,5 heure mais la stabilité est réduite à environ 30 minutes après distribution en seringue.

Le contrôle scintigraphique réalisé après injection intraveineuse de la préparation chez le rat et le lapin ne met pas en évidence de fixation thyroïdienne significative de 99m Technetium libre dans les 3 heures suivant l'injection. La préparation de D 25 marquée est exempte de pyrogènes d'après les tests réalisés in vivo chez le lapin.

### EXEMPLE 4 ADMINISTRATION PAR INHALATION DU PRODUIT RADIOMARQUE

Le D 25 ou ses dérivés radiomarqués sont administrés sous forme d'un aérosol dans des conditions de nébulisation devant respecter la stabilité du marquage et générer des particules de taille compatible avec un rendement de transfert satisfaisant au niveau des alvéoles pulmonaires. Un inhalateur à ultrasons TV 6000 (SIEMENS) opérant à une fréquence de 100 KHz peut être préférentiellement utilisé. La solution est nébulisée à raison de 0,5 ml par minute. L'inhalation est réalisée pendant 15 minutes dans une hotte spéciale entourant la tête du patient pour prévenir toute contamination de l'air par la fraction d'aérosol non inhalée ou partiellement exhalée. Le contrôle par scintigraphie dynamique réalisé sur les poumons au cours de l'inhalation a permis d'évaluer le transfert effectif du produit marqué dans le poumon à 5 - 7,5 % de la dose initiale.

### EXEMPLE 5 OBTENTION DES IMAGES SCINTIGRAPHIQUES

Les scintigraphies sont réalisées en pathologie expérimentale comme chez les patients 2,3 et 4 heures après l'inhalation. Une gamma camera réglée sur le pic photoélectrique de 140 Kev du 99m Technetium est placée au niveau des zones d'intérêt, en incidence antérieure et postérieure. L'acquisition des images est réalisée préférentiellement avec une matrice de 128 x 128 pixels. Les images sont ensuite traitées par informatique pour ajuster le contraste et déterminer le rapport scintigraphique des foyers fixants par rapport à l'activité de zones de références généralement en position contrôlatérale.

### EXEMPLE 6 RESULTATS OBTENUS DANS LA BERYLLIOSE PULMONAIRE EXPERIMENTALE CHEZ LE SINGE

La berylliose expérimentale est un modèle de granulomatose pulmonaire très proche de la sarcoïdose humaine dans ses caractéristiques tant cliniques qu'immunologiques (KRIEBEL D. et al. Am. Rev. Respir. Dis. 1988, 137:464-73) qui a été particulièrement documenté chez l'animal par ANDRE S. (These Doctorat en Biologie, Univ. Paris Val de Marne 1984). Nous avons utilisé des singes Papio papio adultes contaminés depuis 3 à 12 mois par dépôt de Beryllium métal dans le lobe inférieur du poumon droit. Les animaux ont été explorés régulièrement par scintigraphies des fonctions de ventilation (DTPA - Technetium) et perfusion (Macroagregats d'albumine - Technetium) pulmonaires, par radiographie thoracique, tomodensitométrie X et imagerie par résonnance magnétique nucléaire.

Les scintigraphies au D 25 ou à ses dérivés d'hémisynthèse étaient réalisées après marquage de 1 mg de produit par 740 MBq de Technetium 99m.

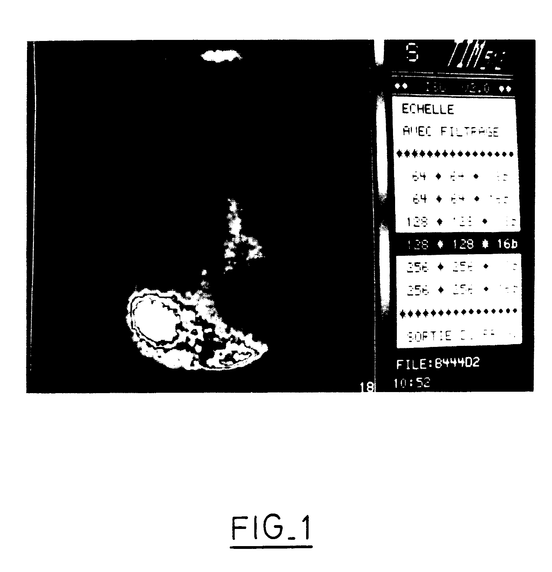

Figure 1 : babouin n° 444, 3 mois après induction d'une bérylliose pulmonaire.
   Scintigraphie au D 25, thorax face postérieure, 3 heures après inhalation.
   On observe une hyperfixation du lobe inférieur du poumon droit présentant une alvéolite (1er stade de la pathologie).
Figure 2 :
   2a : babouin n° 482, 1 an après induction d'une bérylliose pulmonaire.
      Scintigraphie au D 25 - thorax face postérieure - 2 heures après inhalation.
      On observe une fixation paratrachéale droite localisée au niveau des ganglions paratrachéaux rétrocarénaires (2ème stade de la pathologie).
   2b : pièce d'autopsie montrant la présence des ganglions rétrocarénaires inflammatoires de 8 x 3 mm induits par la bérylliose, du territoire fixant à la scintigraphie au D 25.

### EXEMPLE 7 RESULTATS OBTENUS EN PATHOLOGIE HUMAINE

Le D 25 est utilisé à la dose de 1 mg après marquage par 1110 MBq de Technetium.

### a) Dans la sarcoïdose

6 patients ont été explorés en comparant la scintigraphie au D 25 aérosol à celle du citrate de Gallium 67 injecté par voie i.v.

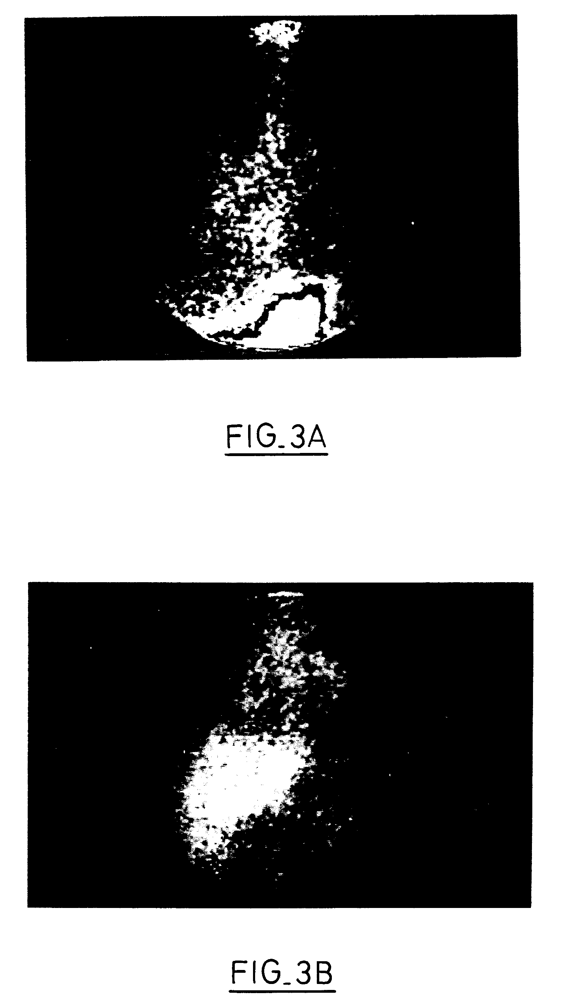

Chez 3 patients présentant une augmentation de la taille des ganglions thoraciques et des foyers réticulonodulaires, l'imagerie scintigraphique des ganglions hilaires avec le D 25, 4 heures après l'inhalation (Fig. 3A), est identique à celle du Gallium, 48 heures après l'injection i.v. (Fig. 3B).

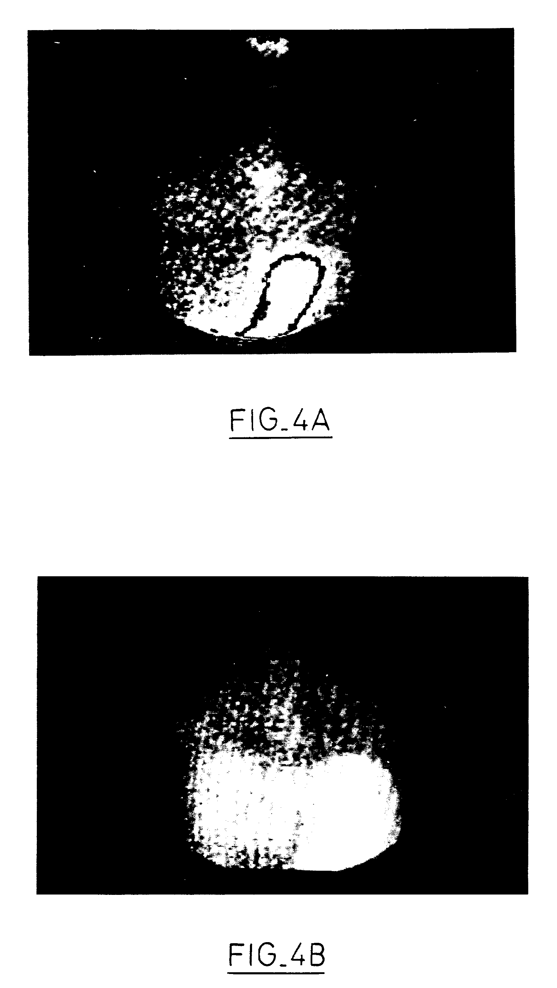

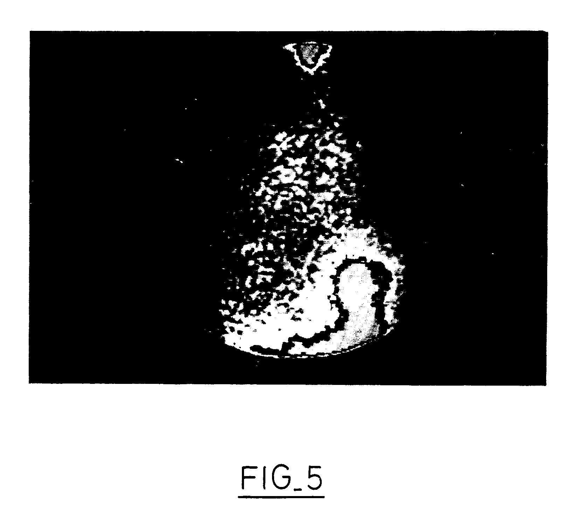

Chez 3 autres patients présentant une pathologie pulmonaire diffuse sans anomalies ganglionnaires visibles en tomodensitométrie X, la scintigraphie au D 25 montre une hyperactivité diffuse des poumons (Fig. 4A) alors que la scintigraphie au Gallium présente un contraste scintigraphique nettement plus faible dans les mêmes zones d'intérêt (Fig. 4B) que la pathologie soit observée au stade II intermédiaire ou dans une phase de fibrose évolutive correspondant au stade III (figure 5)..

### b) Dans les tumeurs pulmonaires

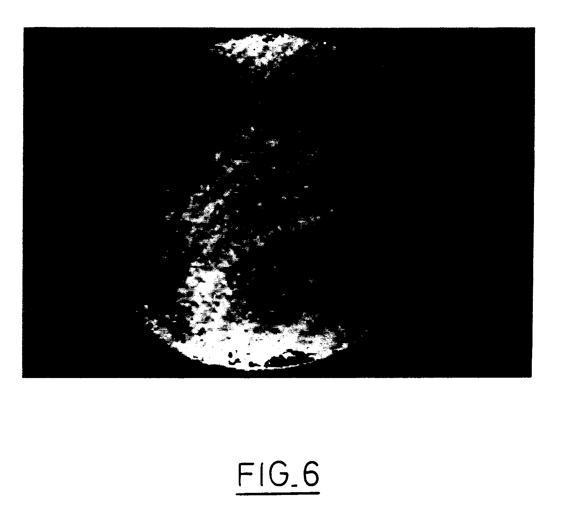

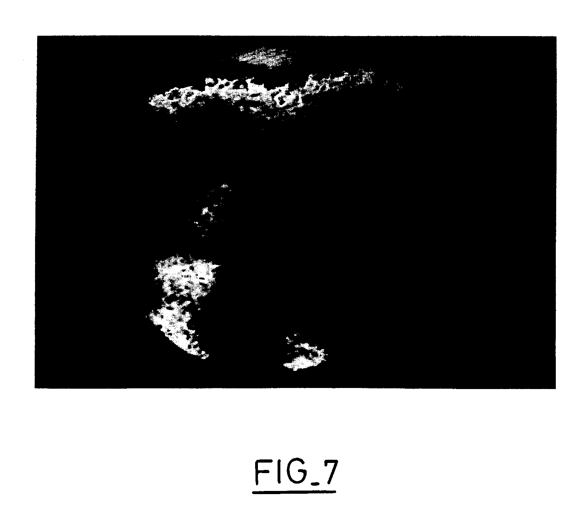

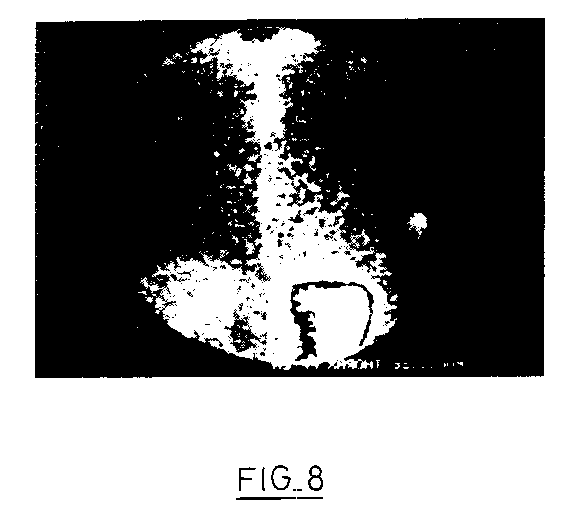

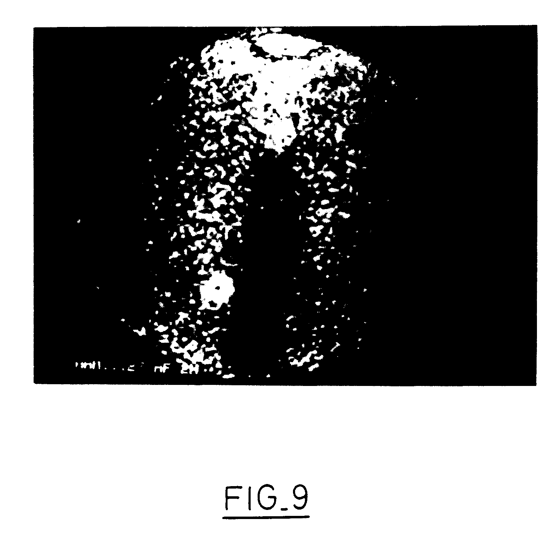

Les scintigraphies au D 25 réalisées sur 15 patients ont mis en évidence chez 8 d'entre eux des fixations significatives au niveau des métastases pulmonaires (figure 6), des tumeurs hilaires, des ganglions médiastinaux et des complexes gangliotumoraux (figure 7). Deux localisations métastatiques périphériques dont une ganglionnaire (figure 8) et l'autre cutanée (figure 9) ont également été détectées. Aucune fixation du D 25 n'a été observée en territoire non pathologique.

### c) Dans les pneumocystoses pulmonaires

4 patients contaminés par le virus VIH et porteurs d'une pneumocystose pulmonaire confirmée par lavage bronchoalvéolaire et non encore traitée ont été explorés par la scintigraphie au D 25 aérosol. Chez 3 d'entre eux, une activité pulmonaire persistant au-delà de 4-5 heures après inhalation a été observée, y compris dans les régions pulmonaires présentant un défaut de ventilation objectivée par la radiographie pulmonaire ou la scintigraphie de la ventilation par inhalation de DTPA marqué au 99m Technetium.
Figure 3 : patient LEV... présentant une sarcoïdose de stade I avec adénopathies médiastinales.
   3a : Scintigraphie au D 25 aérosol, 4 heures après inhalation, thorax en face antérieure. Fixation médiastinale associée à une activité pulmonaire diffuse.
   3b : Scintigraphie au citrate de Gallium, 48 heures après injection intraveineuse. Thorax face antérieure (fixation médiastinale + activité pulmonaire).
Figure 4 : Patient NAR... présentant une sarcoïdose de stade II.
   4a : scintigraphie au D 25 aérosol 4 h 30 après inhalation du thorax en face antérieure (fixation des 2 poumons, plus marquée dans les bases);
   4b : Scintigraphie au citrate de Gallium, thorax face antérieure, 24 heures après injection intraveineuse.
Figure 5 : Patient DJE... présentant une sarcoïdose de stade III avec fibrose évolutive et lésions inflammatoires.
   Scintigraphie au D 25 aérosol, 4 heures après inhalation, du thorax en face antérieure montrant une fixation très marquée sur l'ensemble du territoire pulmonaire.
Figure 6 : Patient TEL... : métastases pulmonaires bilatérales (lâcher de ballons) d'un cylindrome du massif facial.
   La scintigraphie au D 25 aérosol (thorax face postérieure) 4 h 30 après inhalation révèle une hyperactivité du traceur dans les deux poumons.
Figure 7 : Patient NEU... : carcinome épidermoïde du lobe inférieur gauche. 4 heures après inhalation de D 25, la scintigraphie du thorax en face postérieure présente un foyer de fixation intense dans le territoire tumoral.
Figure 8 : Patient ROC... : métastase d'un adénocarcinome à grandes cellules du lobe supérieur du poumon droit.
   Détection par la scintigraphie au D 25, 4 heures après inhalation d'une localisation ganglionnaire axillaire confirmée histologiquement.
Figure 9 : Patient HAM... : métastase d'un adénocarcinome du poumon gauche. Détection par la scintigraphie au D 25 (cuisses face antérieure), 4 heures après inhalation, d'une métastase sous cutanée de taille inférieure à 5 mm dans la région antérointerne de la cuisse droite.

## Revendications (Revendications pour l'(les) Etat(s) contractant(s) suivant(s): AT, BE, CH, DE, DK, ES, FR, GB, IT, LI, LU, NL, SE)

1. Composition aérosol comportant un agent vecteur capable de reconnaître et se fixer sur des foyers inflammatoires et tumoraux mobilisant dans leur environement proche des macrophages qui consiste en un composé polysaccharidique choisi parmi le composé D 25 , le composé D 25 oxydé dans lequel le résidu galacto furanose (Gal_{f}) de la chaîne polysaccharidique linéaire du D 25 a été transformé en arabinose, et leurs dérivés de type amide, ester ou éther, ainsi que leurs sels et dérivés d'ammonium quaternaires, ainsi que les dérivés obtenus par greffage de phosphatides, le dit agent vecteur étant associé à un substrat aérosol

2. Composition aérosol selon la revendication 1, caractérisée en ce que ledit composé est le D 25 de poids moléculaire de 30 ± 10 Kd extrait des protéoglycanes membranaires de la bactérie Klebsiella pneumoniae.

3. Composition aérosol d'imagerie ou de diagnostic in vivo ou ex vivo pour la mise en évidence de foyers inflammatoires et tumoraux mobilisant dans leur environnement des macrophages selon la revendication 1 ou 2, caractérisée en ce qu'elle comporte ledit composé polysaccharidique ou ses dérivés.

4. Composition selon l'une des revendications 1 à 3, caractérisée en ce que ledit composé polysaccharidique ou un de ses dérivés est marqué par un élément détectable tel qu'un élément radioactif, paramagnétique ou fluorescent.

5. Composition selon l'une des revendications 1 à 4, caractérisée en ce qu'elle comporte ledit composé polysaccharidique ou un de ses dérivés non marqués et en ce que ladite composition fait partie d'un kit d'imagerie ou de diagnostic comportant une deuxième composition comprenant une substance capable de reconnaître ledit composé polysaccharidique ou un de ses dérivés, notamment un anticorps, ladite substance étant elle-même marquée par un élément détectable tel qu'un élément radioactif, paramagnétique ou fluorescent.

6. Composition aérosol d'imagerie in vivo selon l'une des revendications 1 à 5, caractérisée en ce que l'élément radioactif est un radionucléide détectable par scintigraphie.

7. Composition selon la revendication 6, caractérisée en ce que le radionucléide est le technetium Tc^{99m}, l'iode¹²³ ou l'indium¹¹¹.

8. Composition d'imagerie ou de diagnostic in vivo selon l'une des revendications 1 à 7, caractérisée en ce qu'elle est utile pour la détection de la sarcoïdose, des alvéolites, des granulomatoses et des pneumocystoses, des pathologies intersticielles du poumon et des lésions inflammatoires et infectieuses.

9. Composition aérosol de diagnostic in vivo selon l'une des revendications 1 à 7 pour l'étude scintigraphique des fonctions de ventilation du poumon et de l'absorption alvéolocapillaire.

10. Composition aérosol pour la thérapie ciblée des foyers inflammatoires et tumoraux mobilisant dans leur proche environnement des macrophages selon la revendication 1 ou 2, caractérisée en ce qu'elle comporte ledit composé polysaccharidique choisi parmi le D 25 et ses dérivés, ledit composé étant couplé à un principe actif anti-inflammatoire ou antitumoral administrable par voie aérosol.

11. Utilisation d'un composé polysaccharidique choisi parmi le composé D 25, le composé D 25 oxydé dans lequel le résidu galacto furanose (Galf) de la chaîne polysaccharidique linéaire du D 25 a été transformé en arabinose, et leurs dérivés de type amide, ester ou éther, ainsi que leurs sels et dérivés d'ammonium quaternaires, ainsi que les dérivés obtenus par greffage de phosphatides.pour la préparation d'une composition destinée à l'administration par inhalation selon l'une des revendications 1 à 10.

## Revendications (Revendications pour l'(les) Etat(s) contractant(s) suivant(s): ES, GR)

1. Utilisation d'un composé polysaccharidique choisi parmi le composé D 25, le composé D 25 oxydé dans lequel le résidu galacto furanose (Gal_{f}) de la chaîne polysaccharidique linéaire du D 25 a été transformé en arabinose, et leurs dérivés de type amide, ester ou éther, ainsi que leurs sels et dérivés d'ammonium quaternaires, ainsi que les dérivés obtenus par greffage de phosphatides pour la préparation d'une composition aérosol destinée à l'administration par inhalation.

2. Utilisation selon la revendication 1, caractérisée en ce que le dit composé est le D 25 de poids moléculaire de 30 ± 10 Kd extrait des protéoglycanes membranaires de la bactérie Klebsiella pneumoniae.

3. Utilisation selon la revendication 1 ou 2 pur la préparation d'une composition aérosol d'imagerie ou de diagnostic in vivo ou ex vivo pour la mise en évidence de foyers inflammatoires et tumoraux mobilisant dans leur environnement des macrophages.

4. Utilisation selon l'une des revendications 1 à 3, caractérisée en ce que le dit composé polysaccharidique ou un de ses dérivés est marqué par un élément détectable tel qu'un élément radioactif, paramagnétique ou fluorescent.

5. Utilisation selon l'une des revendications 1 à 4, caractérisée en ce que la dite composition comporte le dit composé polysaccharidique ou un de ses dérivés non marqués et en ce que la dite composition fait partie d'un kit d'imagerie ou de diagnostic comportant une deuxième composition comprenant une substance capable de reconnaître le dit composé polysaccharidique ou un de ses dérivés, notamment un anticorps, la dite substance étant elle-même marquée par un élément détectable tel qu'un élément radioactif, paramagnétique ou fluorescent.

6. Utilisation selon l'une des revendications 1 à 5 pour la préparation d'une composition aérosol d'imagerie in vivo, caractérisée en ce que l'élément radioactif est un radionucléide détectable par scintigraphie.

7. Utilisation selon la revendication 6, caractérisée en ce que le radionucléide est le technetium Tc^{99m}, l'iode¹²³ ou l'indium¹¹¹.

8. Utilisation selon l'une des revendications 1 à 7 pour la préparation d'une composition d'imagerie ou de diagnostic in vivo utile pour la détection de la sarcoïdose, des alvéolites, des granulomatoses et des pneumocystoses, des pathologies intersticielles du poumon et des lésions inflammatoires et infectieuses.

9. Utilisation selon l'une des revendications 1 à 7 pour la préparation d'une composition aérosol de diagnostic in vivo pour l'étude scintigraphique des fonctions de ventilation du poumon et de l'absorption alvéolocapillaire.

10. Utilisation selon l'une des revendications 1 ou 2 pour la préparation d'une composition aérosol pour la thérapie ciblée des foyers inflammatoires et tumoraux mobilisant dans leur proche environnement des macrophages, caractérisée en ce qu'elle comporte le dit composé polysaccharidique choisi parmi le D 25 et ses dérivés, le dit composé étant couplé à un principe actif anti-inflammatoire ou antitumoral administrable par voie aérosol.

## Patentansprüche (Patentansprüche für folgende(n) Vertragsstaat(en): AT, BE, CH, DE, DK, ES, FR, GB, IT, LI, LU, NL, SE)

1. Aerosol-Zusammensetzung, die ein Vektor-Agens enthält, das erkennen kann und fixiert werden kann an Inflammations- und Tumor-Herden, die in ihrer nahen Umgebung Makrophagen mobilisieren, das besteht aus einer Polysaccharid-Verbindung, ausgewählt aus der Verbindung D 25, der oxidierten Verbindung D 25, in welcher der Galactofuranose-Rest (Gal_{f}) der linearen Polysaccharidkette von D 25 in Arabinose umgewandelt worden ist, und ihren Derivaten vom Amid-, Ester- oder Äther-Typ sowie ihren Salzen und quaternären Ammoniumderivaten sowie den Derivaten, die durch Aufpfropfen von Phosphatiden erhalten worden sind, wobei das genannte Vektor-Agens mit einem Aerosol-Substrat assoziiert ist.

2. Aerosol-Zusammensetzung nach Anspruch 1, dadurch gekennzeichnet, daß es sich bei der genannten Verbindung um die Verbindung D 25 mit einem Molekulargewicht von 30 ± 10 Kd handelt, die aus den Membran-Proteoglycanen des Bakteriums Klebsiella pneumoniae extrahiert worden ist.

3. Aerosol-Zusammensetzung für die in vivo- oder ex vivo-Bilderzeugung oder -Diagnose zum Nachweis von Inflammations- und Tumor-Herden, die in ihrer Umgebung Makrophagen mobilisieren, nach Anspruch 1 oder 2, dadurch gekennzeichnet, daß sie die genannte Polysaccharidverbindung oder ihre Derivate enthält.

4. Zusammensetzung nach einem der Ansprüche 1 bis 3, dadurch gekennzeichnet, daß die genannte Polysaccharid-Verbindung oder eines ihrer Derivate durch ein nachweisbares Element, wie z.B. ein radioaktives, paramagnetisches oder fluoreszierendes Element, markiert ist.

5. Zusammensetzung nach einem der Ansprüche 1 bis 4, dadurch gekennzeichnet, daß sie die genannte Polysaccharidverbindung oder eines ihrer nicht-markierten Derivate enthält und daß die genannte Zusammensetzung Teil eines Bilderzeugungs- oder Diagnose-Kits ist, der eine zweite Zusammensetzung umfaßt, die eine Substanz enthält, welche die genannte Polysaccharid-Verbindung oder eines ihrer Derivate, insbesondere einen Antikörper, erkennen kann, wobei die genannte Substanz selbst durch ein nachweisbares Element, z.B. ein radioaktives, paramgnetisches oder fluoreszierendes Element, markiert ist.

6. Aerosol-Zusammensetzung für die in vivo-Bilderzeugung nach einem der Ansprüche 1 bis 5, dadurch gekennzeichnet, daß das radiaktive Element ein durch Szintigraphie nachweisbares Radionucleid ist.

7. Zusammensetzung nach Anspruch 6, dadurch gekennzeichnet, daß es sich bei dem Radionucleid um Technetium Tc^{99m} Jod¹²³ oder Indium¹¹¹ handelt.

8. Zusammensetzung für die in vivo-Bilderzeugung oder - Diagnose nach einem der Ansprüche 1 bis 7, dadurch gekennzeichnet, daß sie verwendbar ist für den Nachweis der Sarcoidose, der Alveoliten, der Granulomatosen und der Pneumocystosen, der interstitiellen pathologischen Zustände der Lunge und der inflammatorischen und infektiösen Verletzungen.

9. Aerosol-Zusammensetzung für die in vivo-Diagnose nach einem der Ansprüche 1 bis 7 für die szintigraphische Untersuchung der Atmungsfunktionen der Lunge und der Alveolkapillar-Absorption.

10. Aerosol-Zusammensetzung für die gezielte Therapie der Inflammations- und Tumorherde, die in ihrer nahen Umgebung Makrophagen mobilisieren, nach Anspruch 1 oder 2, dadurch gekennzeichnet, daß sie die genannte Polysaccharidverbindung enthält, die ausgewählt wird aus der Verbindung D 25 und ihren Derivaten, wobei diese Verbindung an einen antiinflammatorischen oder Antitumorwirkstoff gekoppelt ist, der als Aerosol verabreicht werden kann.

11. Verwendung einer Polysaccharid-Verbindung, ausgewählt aus der Verbindung D 25, der oxidierten Verbindung D 25, in welcher der Galactofuranose-Rest (Galf) der linearen Polysaccharid-Kette von D 25 in Arabinose umgewandelt worden ist, und ihrer Derivate vom Amid-, Ester- oder Äther-Typ sowie ihrer Salze und quaternären Ammoniumderivate sowie ihrer Derivate, die erhalten wurden durch Aufpfropfung von Phosphatiden, zur Herstellung einer Zusammensetzung nach einem der Ansprüche 1 bis 10, die für die Verabreichung durch Inhalation bestimmt ist.

## Patentansprüche (Patentansprüche für folgende(n) Vertragsstaat(en): ES, GR)

1. Verwendung einer Polysaccharid-Verbindung, die ausgewählt wird aus der Verbindung D 25, der oxidierten Verbindung D 25, in welcher der Galactofuranose-Rest (Gal_{f}) der linearen Polysaccharid-Kette von D 25 in Arabinose umgewandelt worden ist, und ihren Derivaten vom Amid-, Ester- oder Äther-Typ, sowie ihrer Salze und quaternären Ammoniumderivate sowie ihrer Derivate, die durch Aufpfropfen von Phosphatiden erhalten wurden, für die Herstellung einer Aerosol-Zusammensetzung, die für die Verabreichung durch Inhalation bestimmt ist.

2. Verwendung nach Anspruch 1, dadurch gekennzeichnet, daß es sich bei der genannten Verbindung um die Verbindung D 25 mit einem Molekulargewicht von 30 ± 10 Kd handelt, die aus den Membran-Proteoglycanen des Bakteriums Klebsiella pneumoniae extrahiert worden ist.

3. Verwendung nach Anspruch 1 oder 2 zur Herstellung einer Aerosol-Zusammensetzung für die in vivo- oder ex vivo-Bilderzeugung oder -Diagnose zum Nachweis von Inflammations- und Tumor-Herden, die in ihrer Umgebung Makrophagen mobilisieren.

4. Verwendung nach einem der Ansprüche 1 bis 3, dadurch gekennzeichnet, daß die genannte Polysaccharid-Verbindung oder eines ihrer Derivate durch ein nachweisbares Element, z.B. ein radioaktives, paramagnetisches oder fluoreszierendes Element markiert ist.

5. Verwendung nach einem der Ansprüche 1 bis 4, dadurch gekennzeichnet, daß die genannte Zusammensetzung die genannte Polysaccharidverbindung oder eines ihrer nicht-markierten Derivate enthält und daß die genannte Zusammensetzung Teil eines Bilderzeugungs- oder Diagnose-Kits ist, der eine zweite Zusammensetzung umfaßt, die eine Substanz enthält, welche die genannte Polysaccharid-Verbindung oder eines ihrer Derivate, insbesondere einen Antikörper, erkennen kann, wobei die genannte Substanz selbst durch ein nachweisbares Element, z.B. ein radioaktives, paramgnetisches oder fluoreszierendes Element markiert ist.

6. Verwendung nach einem der Ansprüche 1 bis 5 zur Herstellung einer Aerosol-Zusammensetzung für die in vivo-Bilderzeugung, dadurch gekennzeichnet, daß das radiaktive Element ein durch Szintigraphie nachweisbares Radionucleid ist.

7. Verwendung nach Anspruch 6, dadurch gekennzeichnet, daß es sich bei dem Radionucleid um Technetium Tc^{99m}, Jod¹²³ oder Indium¹¹¹ handelt.

8. Verwendung nach einem der Ansprüche 1 bis 7 zur Herstellung einer Zusammensetzung für die in vivo-Bilderzeugung oder -Diagnose, die verwendbar ist für den Nachweis der Sarcoidose, der Alveoliten, der Granulomatosen und der Pneumocystosen, der interstitiellen pathologischen Zustände der Lunge und der inflammatorischen und infektiösen Verletzungen.

9. Verwendung nach einem der Ansprüche 1 bis 7 zur Herstellung einer Aerosol-Zusammensetzung für die in vivo-Diagnose für die szintigraphische Untersuchung der Atmungsfunktionen der Lunge und der Alveolkapillar-Absorption.

10. Verwendung nach einem der Ansprüche 1 oder 2 zur Herstellung einer Aerosol-Zusammensetzung für die gezielte Therapie der Inflammations- und Tumorherde, die in ihrer nahen Umgebung Makrophagen mobilisieren, dadurch gekennzeichnet, daß sie die genannte Polysaccharidverbindung enthält, die ausgewählt wird aus der Verbindung D 25 und ihren Derivaten, wobei diese Verbindung an einen antiinflammatorischen oder Antitumor-Wirkstoff gekoppelt ist, der als Aerosol verabreicht werden kann.

## Claims (Claims for the following Contracting State(s): AT, BE, CH, DE, DK, ES, FR, GB, IT, LI, LU, NL, SE)

1. Aerosol composition containing a carrier agent capable of recognizing and binding to inflammatory and tumour foci mobilizing macrophages in their immediate environment which consists of a polysaccharide compound selected from the compound D 25, the compound oxidized D 25 in which the galactofuranose residue (Gal_{f}) of the linear polysaccharide chain of D 25 has been converted to arabinose, and their amide, ester or ether type derivatives, as well as their quaternary ammonium derivatives and salts, and also the derivatives obtained by grafting of phosphatides, said carrier agent being associated to an aerosol substrate .

2. Aerosol composition according to claim 1, characterized in that the said compound is D 25 of molecular weight 30 ± 10 kD extracted from the membrane proteoglycans of the bacterium Klebsiella pneumoniae.

3. Aerosol composition for in vivo or ex vivo imaging or diagnosis, for detecting inflammatory and tumour foci mobilizing macrophages in their environment according to Claim 1 or 2, characterized in that it contains the said polysaccharide compound or its derivatives.

4. Composition according to one of Claims 1 to 3, characterized in that the said polysaccharide compound or one of its derivatives is labelled with a detectable component such as a radioactive, paramegnetic or fluorescent component.

5. Composition according to one of Claims 1 to 4, characterized in that it contains the said polysaccharide compound or one of its derivatives unlabelled, and in that the said composition forms part of an imaging or diagnostic kit containing a second composition comprising a substance capable of recognizing the said polysaccharide compound or one of its derivatives, in particular an antibody, the said substance itself being labelled with a detectable component such as a radioactive, paramagnetic or fluorescent component.

6. Aerosol composition for in vivo imaging according to one of claims 1 to 5, characterized in that the radioactive component is a radionuclide detectable by scintigraphy.

7. Composition according to claim 6, characterized in that the radionuclide is technetium^{99m} Tc ¹²³ iodine or¹¹¹indium.

8. Composition for in vivo imaging or diagnosis according to one of claims 1 to 7, characterized in that it is useful for the detection of sarcoïdosis, alveolitis, granulomatosis, pneumocystosis, lung intersticial pathology, inflammatory and infectious lesions.

9. Aerosol composition for in vivo diagnosis according to one of claims 1 to 7 for the scintigraphic study of lung ventilatory functions and of the alveocapillary absorption.

10. Aerosol composition for the targeted therapy of inflammatory and tumour foci mobilizing macrophages in their immediate environment according to claim 1 or 2, characterized in that it contains the said polysaccharide compound selected from D 25 and its derivatives, the said compound being coupled to an anti-inflammatory or anti-tumour active principle, administrable in the form of an aerosol .

11. Use of a polysaccharide compound selected from the compound D 25, the compound oxidized D 25 in which the galactofuranose residue (Gal_{f} ) of the linear polysaccharide chain of D 25 has been converted to arabinose, and their amide, ester or ether derivatives, as well as their quaternary ammonium derivatives and salts, and also the derivatives obtained by grafting of phosphatides, for preparing a composition designed to administration by inhalation according to anyone of claims 1 to 10.

## Claims (Claims for the following Contracting State(s): ES, GR)

1. Use of a polysaccharide compound selected from the compound D 25, the compound oxidized D 25 in which the galactofuranose residue (Gal_{f}) of the linear polysaccharide chain of D 25 has been converted to arabinose, and their amide, ester or ether type derivatives, as well as their quaternary ammonium derivatives and salts, and also the derivatives obtained by grafting of phosphatides, for preparing an aerosol composition designed to administration by inhalation.

2. Use according to claim 1, characterized in that the said compound is D 25 of molecular weight 30 ± 10 kD extracted from the membrane proteoglycans of the bacterium Klebsiella pneumoniae.

3. Use according to claim 1 or 2 for preparing an aerosol composition for in vivo or ex vivo imaging or diagnosis, for detecting inflammatory and tumour foci mobilizing macrophages in their environment.

4. Use according to one of claims 1 to 3, characterized in that the said polysaccharide compound or one of its derivatives is labelled with a detectable component such as a radioactive, paramagnetic or fluorescent component.

5. Use according to one of claims 1 to 4, characterized in that it contains the said polysaccharide compound or one of its derivatives unlabelled, and in that the said composition forms part of an imaging or diagnostic kit containing a second composition comprising a substance capable of recognising the said polysaccharide compound or one of its derivatives, in particular an antibody, the said substance itself being labelled with a detectable component such as a radioactive, paramagnetic or fluorescent component.

6. Use according to anyone of claims 1 to 5 for preparing an aerosol composition for in vivo imaging, characterized in that the radioactive component is a radionuclide detectable by scintigraphy.

7. Use according to claim 6, characterized in that the radionuclide is technetium ^{99m}Tc, ¹²³iodine or ¹¹¹indium.

8. Use according to anyone of claims 1 to 7 for preparing a composition for in vivo imaging or diagnosis, characterized in that it is useful for the detection of sarcoidosis, alveolitis, granulomatosis, pneumocystosis, lung intersticial pathology, inflammatory and infectious lesions.

9. Use according to anyone of claims 1 to 7 for preparing an aerosol composition for in vivo diagnosis for the scintigraphic study of lung ventilatory functions and of the alveocapillary absorption.

10. Use according to anyone of claims 1 or 2 for preparing an aerosol composition for the targeted therapy of inflammatory and tumour foci mobilizing macrophages in their immediate environment characterized in that it contains the said polysaccharide compound selected from D 25 and its derivatives, the said compound being coupled to an anti-inflammatory or anti-tumour active principle , administrable in the form of an aerosol.
